# FASCICULE DE BREVET EUROPEEN

(11) **EP 1 191 905 B1**
(45) Date de publication et mention de la délivrance du brevet: **27.09.2006**
(21) Numéro de dépôt: 00936959.6
(22) Date de dépôt: 29.05.2000
(51) Int. Cl.: A61F 2/12

(54) **PROTHESE MAMMAIRE**
BRUSTPROTHESE
BREAST PROSTHESIS

(30) Priorité: 02.06.1999 FR 9906929
(43) Date de publication de la demande: 03.04.2002
(73) Titulaire: Missana, Marie-Christine, 94210 La Varenne Saint Hilaire (FR); Rochebiliere, Arnaud, 83000 Toulon (FR)
(72) Inventeur: Missana, Marie-Christine, 94210 La Varenne Saint Hilaire (FR); Rochebiliere, Arnaud, 83000 Toulon (FR)
(74) Mandataire: Roman, Michel
(86) Numéro de dépôt international: PCT/FR2000/001457
(87) Numéro de publication internationale: WO 2000/074599

(56) Documents cités:
- EP-A- 0 115 384
- WO-A-96/40003
- FR-A- 2 726 173
- GB-A- 2 040 688
- GB-A- 2 136 692
- US-A- 2 651 783
- US-A- 3 665 520
- US-A- 5 066 302

## Description

L'invention concerne une prosthèse mammaire implantable plus particulièrement destinée à la chirurgie d'augmentation mammaire et à la chirurgie reconstructrice mammaire.

Les prothèses mammaires sont généralement constituées d'une poche souple en élastomère du type silicone que l'on remplit avec un fluide plus ou moins visqueux. En Europe, ce fluide est le plus souvent à base de sérum physiologique que l'on introduit dans la poche au cours de l'implantation de la prothèse par une ouverture appropriée dans la poche rendue étanche après remplissage. Aux États-Unis notamment, on a aussi recours à des gels de silicone qui sont introduits dans la poche ensuite fermée hermétiquement avant implantation. Un type particulier de prothèse est appelé prothèse d'expansion : elles sont implantées sous la zone de tissus à expandre puis sont progressivement remplies par un système de valve approprié d'un fluide de type sérum physiologique. On trouvera dans les demandes de brevet FR-2 735 354, FR-2 726 173 et FR-2 667 539 quelques exemples de réalisation de ces prothèses classiques ou d'expansion.

De nombreuses prothèses disponibles sur le marché adoptent des formes rondes, dites "haut profil", "bas profil" ou encore "anatomiques", comme la prothèse objet du brevet US-3 665 520 formée d'une poche flexible asymétrique comportant une paroi frontale ayant approximativement la forme du sein humain et une paroi arrière ayant approximativement celle de la paroi du thorax. Toutes ces prothèses tentent de s'approcher au mieux de la forme du sein, mais aucune n'y parvient pleinement. Les prothèses rondes manquent de naturel, sont trop saillantes dans les quadrants supérieurs et internes (de façon connue, on peut décomposer le volume du sein - et donc de la prothèse - en quatre quadrants en fonction de leur position par rapport au buste). Quant aux formes dites "anatomiques, elles présentent une forme plus adaptée mais elles peuvent facilement être mal positionnée dans la loge chirurgicale car leurs faces postérieures (c'est-à-dire la zone de la prothèse qui va être placée en contact avec le thorax que l'on appelle également base d'implantation) ont un contact insuffisant avec le thorax.

La présente invention a pour but l'amélioration dans la conception des prothèses mammaires, amélioration visant notamment un aspect plus esthétique, plus proche de celui du sein naturel quelle que soit la position assise, debout ou couchée de la personne portant la prothèse, et/ou une plus grande facilité à positionner correctement la prothèse lors de l'implantation et/ou un maintien plus constant de la prothèse une fois implantée en position correcte et/ou un port de la prothèse plus agréable.

L'invention a tout d'abord pour objet une prothèse mammaire selon la revendication 1.

On entend au sens de l'invention par « latéralisée » le fait que la poche une fois remplie délimite un volume que l'on ne peut poser indifféremment à gauche ou à droite sur le thorax de la personne : on a donc une prothèse droite et une prothèse gauche qui sont spécifiques comme le sont les seins naturels, notamment par la géométrie de la face postérieure de la poche et/ou celle de la face antérieure de celle-ci (« antérieure » désigne, par opposition à « postérieure », la surface développée par la poche tournée du côté opposé au thorax).

Outre cette latéralisation se traduisant par des différences dans la géométrie entre d'une part les quadrants externes (inférieur et supérieur) et d'autre part les quadrants internes (inférieur et supérieur), on peut prévoir très avantageusement une latéralisation supplémentaire se traduisant par des différences dans la géométrie entre d'une part les quadrants inférieurs (interne et externe) et d'autre part les quadrants supérieurs (interne et externe) : ainsi, on peut également distinguer dans chaque prothèse de l'invention, la partie « haute » et la partie « basse » en position d'implantation qui ne peuvent être interverties.

On entend au sens de l'invention par « poche susceptible de contenir » soit la prothèse complète, avec la poche déjà entièrement remplie du fluide de remplissage, soit la poche partiellement remplie, soit la poche encore vide, puisqu'on a vu plus haut que les prothèses, notamment suivant le type de fluide de remplissage choisi ou la fonction de la prothèse, se trouvent avant implantation sous forme de poches vides ou déjà remplies. De préférence il s'agit d'une prothèse définitive.
La géométrie de la poche déterminant celle de la prothèse dans son ensemble une fois la poche remplie, la définition de l'invention est apparue plus claire en référence à une poche remplie, définissant 1e volume se rapprochant de celui du sein.

Les inventeurs ont en effet pris conscience qu'une telle « latéralisation «, une telle dissymétrie était la clé du problème pour surmonter les inconvénients des prothèses actuellement utilisées.

Cette latéralisation peut être réalisée à différents niveaux, qui peuvent être alternatifs, ou, préférentiellement, cumulatifs.

Ainsi, un premier niveau est le choix d'une asymétrie de la poche en position d'implantation (par convenance, la position d'implantation est celle d'une personne debout ou assise avec le buste droit), une fois remplie, par rapport à un plan P1 passant par le mamelon E (le pôle antérieur de la face antérieure simulant le mamelon du sein) et par les bords antérieurs inférieur D et supérieur B. Cette caractéristique prend ainsi en compte le caractère asymétrique des seins par rapport à un plan vertical sagittal. En effet, un sein naturel n'est hémisphérique que chez l'adolescente. Par la suite, il se déroule sur la paroi thoracique et présente alors progressivement un aspect plus arrondi et plus saillant dans les quadrants inférieurs et externes . L'asymétrie selon l'invention permet avantageusement que le volume du quadrant inférieur externe de la prothèse implantée soit plus important que celui du quadrant inférieur interne et/ou que celui du quadrant supérieur externe soit plus important que celui du quadrant supérieur interne.

De préférence, cette asymétrie est définie par une différence dans les dimensions entre d'une part la projection de la distance EC entre le mamelon et le bord antérieur interne, et d'autre part la projection de la distance EA entre ledit mamelon et le bord antérieur externe, les projections étant faites selon un plan P2 perpendiculaire au plan P1 passant par le mamelon évoqué plus haut et contenant ledit mamelon E ainsi que le bord antérieur supérieur B. Le rapport entre ces deux projections est avantageusement inférieur ou égal à 0,95, notamment compris entre 0,8 et 0,9 ou entre 0,85 et 0,90. Le mode de réalisation préféré consiste à choisir un rapport de l'ordre de 0,875 qui est celui le mieux à même de reproduire l'aspect plus saillant vers l'extérieur du sein naturel, alors que les prothèses actuelles ont un rapport strictement égal à 1.

Par contre, il est préférable que la projection de la distance EC entre le mamelon E et le bord interne C soit égale à ou très voisine de la projection de la distance EA' entre le mamelon E et le bord postérieur externe A', selon ce même plan P2 : cette configuration permet notamment l'obtention d'un « débordement » extérieur de la poche remplie en position d'implantation par rapport à la face postérieure de celle-ci, débordement s'étendant notamment entre les bords postérieurs inférieur D et supérieur B, qui simule au mieux l'aspect du sein naturel.

Avantageusement, la prothèse est conçue de façon à ce que selon le plan P2 décrit plus haut, la dimension de la projection de la distance BE entre le bord supérieur B et le mamelon E est supérieur à la dimension de la projection de la distance ED entre le mamelon et le bord inférieur D. Le rapport r (BE / ED) est de préférence d'au moins 1.1, notamment entre 1.1 et 2 ou entre 1.3 et 1.5.

Incidemment, pour obtenir l'effet de débordement « extérieur » évoqué plus haut, il est avantageux de faire en sorte que le plan P5 tangeant en k (le bord postérieur externe) à la face antérieure de la prothèse fasse en k avec le plan P6 tangeant audit point k à la face postérieure un angle φ obtus, notamment supérieur à 95 ou 100°, notamment compris entre 91° et 120°, par exemple de l'ordre de 115°.

De préférence, la prothèse ne contient pas de prolongement axillaire ou autre.

Un second niveau de latéralisation concerne la prise en compte de la convexité naturelle du thorax dans un plan horizontal (toujours avec la convention que la prothèse est remplie et en position d'implantation) . Les prothèses actuelles ont une face postérieure plane. Les malpositions, rotations prothétiques et disgrâces esthétiques observées après implantation s'expliquent notamment par ce choix : les prothèses n'épousant le plan thoracique que sur une surface insuffisante, elles peuvent facilement se déplacer. Selon l'invention, au contraire, on confère préférentiellement à la face postérieure au moins une courbure concave, afin d'augmenter cette surface de contact et donc d'améliorer l'assise de la prothèse sur le thorax.

Avantageusement, on prévoit une première courbure concave dans un plan P3 horizontal, plan passant par exemple par le bord interne C. Dans ce cas, la projection perpendiculaire GG' du pôle G de la face postérieure sur le plan P4 horizontal et contenant le bord externe postérieur A' et le bord interne C est d'au moins 3 mm, notamment d'au moins 5 mm ou 1 cm, par exemple de 0,8 à 1,5 cm.

Avantageusement encore, la face postérieure peut aussi présenter une courbure concave dans un plan vertical P9 cette fois, plan vertical passant par exemple par le bord supérieur B. Dans ce cas, la projection perpendiculaire HH' du pôle H de la face postérieure selon cette courbure sur un plan P10 vertical, perpendiculaire à P9 et passant par le bord supérieur B est d'au moins 2 mm, notamment comprise entre 3 et 6 mm.

C'est avec cette double courbure que la face postérieure moule le mieux la courbure du thorax. Avantageusement, la première courbure est ininterrompue entre le bord interne C et le bord externe postérieur A', et de même la seconde courbure est également ininterrompue du bord supérieur B au bord postérieur inférieur D (c'est-à-dire sans zone plane ou de courbure inversée entre les deux points considérés pour chacune des courbures).

Pour obtenir cette face postérieure non plane, on peut la rendre plus rigide, moins déformable que la face antérieure, par exemple en épaississant sélectivement l'épaisseur de la paroi de cette face.

Un troisième niveau de latéralisation se rapporte aux zones de « raccordement » entre la prothèse et le thorax : les prothèses actuelles totalement symétriques ne prennent généralement pas non plus en compte le fait que les seins naturels, notamment dans la zone interne et dans la zone supérieure, se raccordent au thorax avec une pente « douce » et non de façon quasi perpendiculaire au thorax.

Selon l'invention, au contraire, on prévoit tout d'abord de simuler ce raccordement en pente douce en zone supérieure de la prothèse, en concevant la prothèse de façon à ce que les plans P10 et P11 tangeant respectivement à la face postérieure et à la face antérieure de la poche une fois remplie et en position d'implantation au bord supérieur B font un angle entre eux inférieur ou égal à 70°, notamment inférieur ou égal à 65 ou 60°, par exemple de l'ordre de 40°.

Alternativement ou cumulativement, l'invention prévoit également un raccordement en pente douce de la prothèse avec le thorax dans la zone interne de la prothèse : la poche est conçue de façon à ce que les plans P7 et P8 tangeant respectivement à la face antérieure et à la face postérieure de la poche une fois remplie et en position d'implantation au bord interne C font entre eux un angle inférieur ou égal à 70°, notamment inférieur ou égal à 65 ou 60°, par exemple de l'ordre de 40°.

L'invention a pour objet les prothèses ayant au moins un niveau de latéralisation et appartenant à la famille des prothèses définies dans le préambule de la présente demande. Elle concerne des prothèses ayant tous les volumes utilisés couramment en chirurgie mammaire, à savoir des prothèses qui, une fois remplies, ont un volume pouvant aller de 80 cm3 à 700 cm3.

Les détails et caractéristiques avantageuses de l'invention vont maintenant ressortir d'un exemple suivant non limitatif, à l'aide des figures 1 à 6 :
- Figure 1 : une coupe transversale schématisée d'un thorax avec des seins naturels
- Figure 2 : la même coupe avec deux prothèses selon l'art antérieur
- Figure 3 : la même coupe avec deux prothèses selon l'invention
- Figure 4 : une vue de la face antérieure selon un plan vertical de la prothèse droite selon l'invention,
- Figure 5 : une vue de la prothèse de la figure 4 en coupe horizontale
- Figure 6 : une vue de la prothèse de la figure 4 de profil

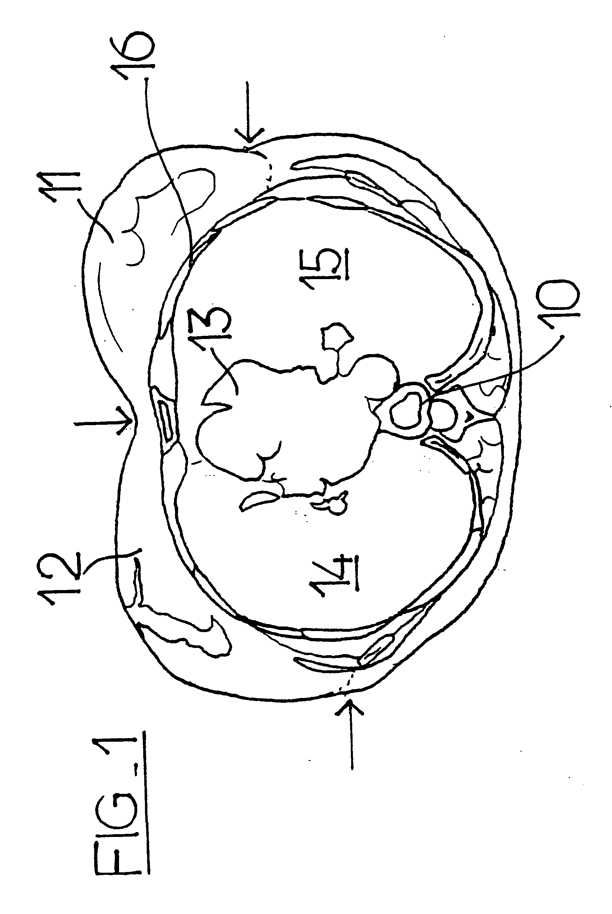

La figure 1 est donc une coupe transversale thoracique en fenêtre médiastinale passant par la quatrième vertèbre dorsale, représentée de façon schématique à partir d'un cliché scannographique. On voit la colonne vertébrale 10, les deux seins 11 et 12, le médiastin 13, les champs pulmonaires 14 et 15, le plan costal 16. On peut constater que les deux seins se « déroulent » naturellement sur le plan thoracique 16 en épousant sa forme convexe. Les flèches représentent les limites internes et externes de la projection sur le thorax des deux glandes mammaires.

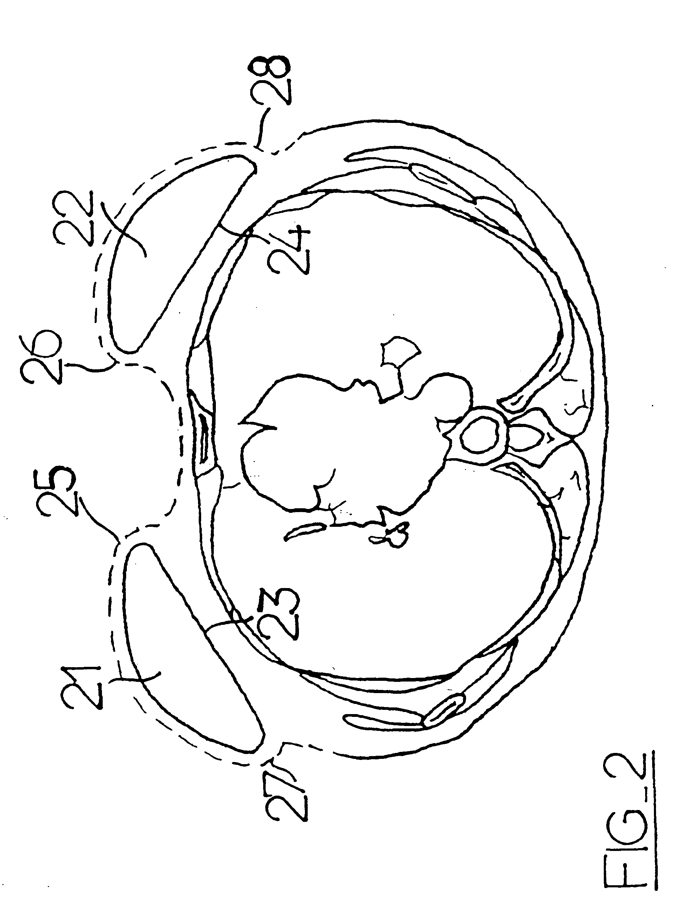

La figure 2 montre, selon la même coupe qu'à la figure 1, certains des inconvénients d'un type de prothèse (comparative) actuellement disponible commercialement : les prothèses 21 et 22 ont des faces postérieures 23 et 24 planes qui ne suivent pas la courbure du thorax. En outre, elles créent dans les zones internes 25 et 26 de raccordement avec le thorax un angle quasiment à 90°avec ledit thorax. Et on est à peu près dans la même situation dans les zones de raccordement externes 27 et 28 : d'une part, l'apparence externe de la prothèse est donc inesthétique, et d'autre part, elle est susceptible de se déplacer dans la loge où elle est implantée, augmentant l'effet inesthétique et l'effet d'inconfort pour la personne.

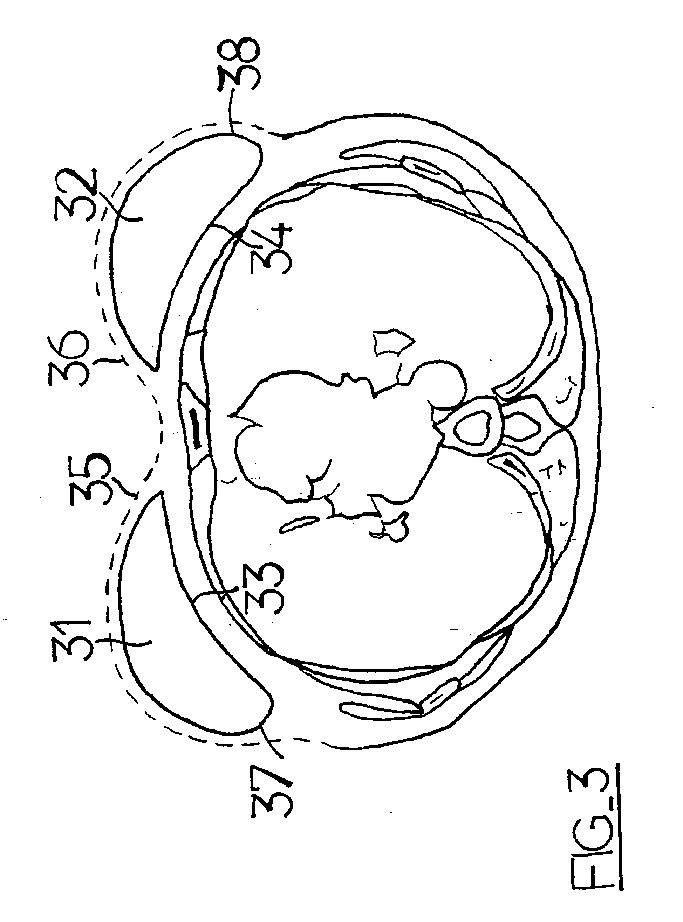

La figure 3 représente les prothèses 31,32 selon un mode de réalisation préféré de l'invention : elles se rapprochent beaucoup plus de l'aspect des seins de la figure 1, avec une face postérieure 33,34 moulant au mieux la convexité du thorax, et des raccordements en zones internes 35, 36 et en zone externe,37,38 en pente douce. Les prothèses 31,32 ont un volume mieux réparti et plus proche de la loge thoracique ; de fait, elles sont donc bien moins susceptibles de se déplacer. On voit également que les prothèses 31,32, contrairement aux prothèses 21,22 ne sont pas interchangeables. Elles sont latéralisées, asymétriques comme le sont les seins naturels.

Les figures suivantes vont détailler la géométrie de la prothèse 31.

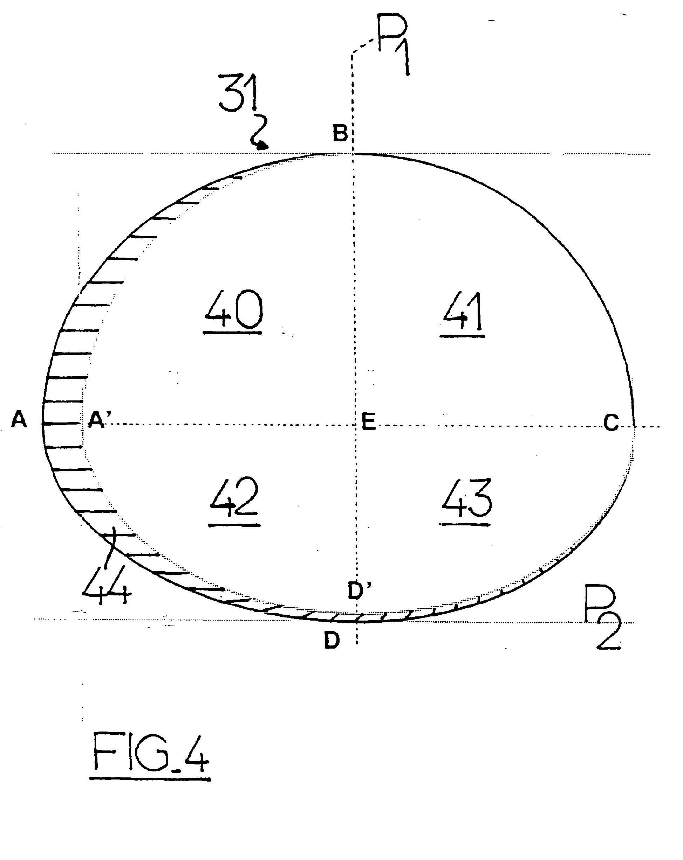

La figure 4 montre donc une vue antérieure de la prothèse droite 31 de la figure 3. Il va de soi que l'on peut déduire de cette représentation, ainsi que toutes les suivantes celles de la prothèse gauche, qui est la construction en miroir en volume de la prothèse droite. Cette représentation et les suivantes sont à l'échelle 1.

Le point E représenté est le pôle antérieur de la prothèse, qui correspond au mamelon du sein naturel, le point C est le bord interne (celui qui va être tourné vers l'autre prothèse en position d'implantation), les points B et D sont respectivement les bords antérieurs supérieur et inférieur, le point A est le bord antérieur externe (par opposition à « interne »), le point A' le bord postérieur externe et le point D' le bord postérieur inférieur. Il s'agit d'une prothèse d'un volume d'environ 480 cm3.

Les dimensions des distances entre ces différents points sont les suivantes, mesurées dans le plan P2 :
AA' = 1 cm ( longueur du débordement externe)
A'C = 14 cm (base de la prothèse)
AC = 15 cm (largeur totale de la prothèse)
BD = 12 cm (hauteur totale de la prothèse)
DD' = 2 mm
A'E = EC = 7cm
AE = 8cm
BE = 7cm
ED = 5cm

On voit que la prothèse ne présente pas de symétrie par rapport au plan P1 passant par B, D et E et perpendiculaire au plan P2 de représentation de la figure : la distance EC est notablement inférieure à la distance AE, et les volumes des quadrants externes supérieurs 40 et inférieur 42 sont plus importants que celui des volumes des quadrants internes supérieur 41 et inférieur 43. On a une zone hachurée 44 qui correspond à un débordement de la face antérieure par rapport à la surface développée par la surface postérieure, ce que traduit la distance séparant les points A et A'. Ce débordement est le plus important à proximité des points A et A', mais on voit qu'il se prolonge jusque dans le quadrant interne inférieur 43 (1a distance entre D et D' n'est pas négligeable). La prothèse présente aussi une dissymétrie entre les volumes des quadrants externes inférieur 42 et supérieur 40 d'une part, et entre les volumes des quadrants internes inférieur 43 et supérieur 41 d'autre part, ce que traduit la différence entre les distances BE et ED. Dans le cas présent, le rapport r (BE/ED) est de 1.4. Ce rapport peut plus généralement être choisi de préférence entre 1.1 et 2, notamment entre 1.3 et 1.5.

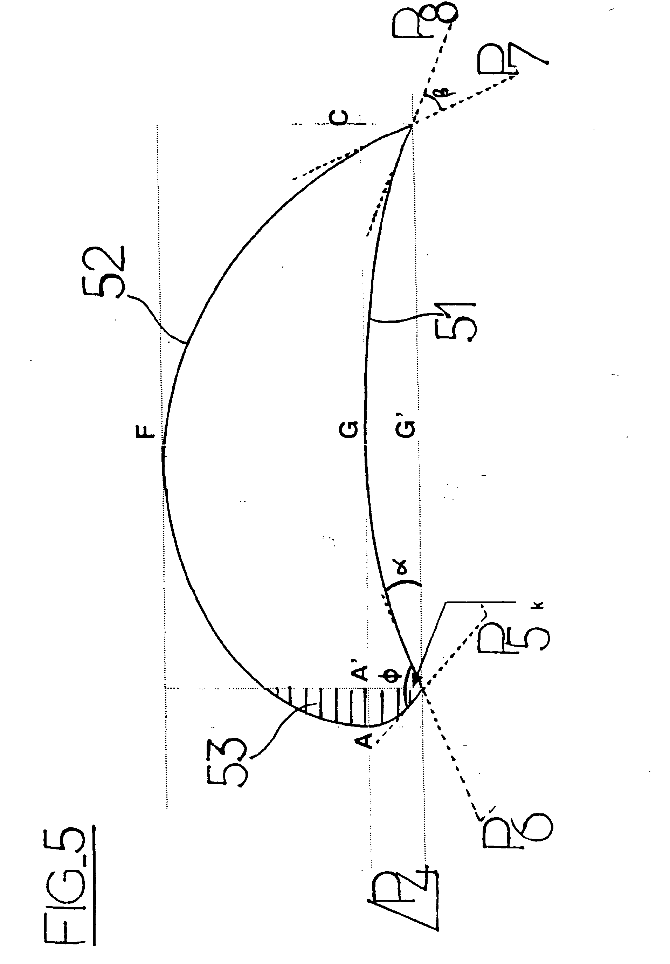

La figure 5 est une vue en coupe horizontale de la représentation précédente. Le point F est selon cette coupe le pôle de la face antérieure 52 et G le pôle de la face postérieure 51. G' est la projection de G dans le plan P4 qui est le plan perpendiculaire au plan de la coupe et qui passe par k et C. A noter que les axes BD de la figure 4 et FG de cette figure sont perpendiculaires l'un à l'autre mais avec un décalage de 1 cm environ. Ils ne se croisent pas.

Les distances entre ces différents points sont :
GG' = 1,3 cm
FG = 5 cm (projection antérieure de la prothèse)
kG' = 6 cm
GC = 8cm

On voit donc facilement que la face postérieure 51 présente une concavité régulière s'étendant entre les points k et C. Cette concavité peut se quantifier par la distance GG' qui est supérieur à 1 cm, et par les angles α que font les plans tangeants à la face postérieure 51 aux points k et C avec le plan P4. Ici les deux angles du côté externe et interne sont sensiblement identiques (d'environ 25°, qui peut être compris entre 20° et 30°), mais cela peut ne pas être le cas . On peut noter que G' n'est pas au milieu de kC. On a un rapport A'G/G'C d'environ 0,75 ( par exemple compris entre 0.5 et 1.).La zone hachurée 53 correspond au débordement externe référencé 44 à la figure précédente elle permet de voir plus clairement que la prothèse permet l'obtention de l'effet naturel d'un sein saillant vers l'extérieur.

La figure 5 permet aussi de représenter un raccordement en pente douce évoqué plus haut, le raccordement entre le bord interne C de la prothèse et le thorax : ainsi, le plan tangeant à la face postérieure 51 au point C fait avec le plan tangeant à la face antérieure au même point C un angle β faible, bien inférieur à 90°, ici de l'ordre de 40°.

La figure 5 montre également que le débordement externe se traduit aussi par un angle φ en k d'environ 115° entre le plan P5 passant par k et tangeant à la face antérieure 52 et le plan P6 passant par k aussi et tangeant à la face postérieure 51 de la prothèse.

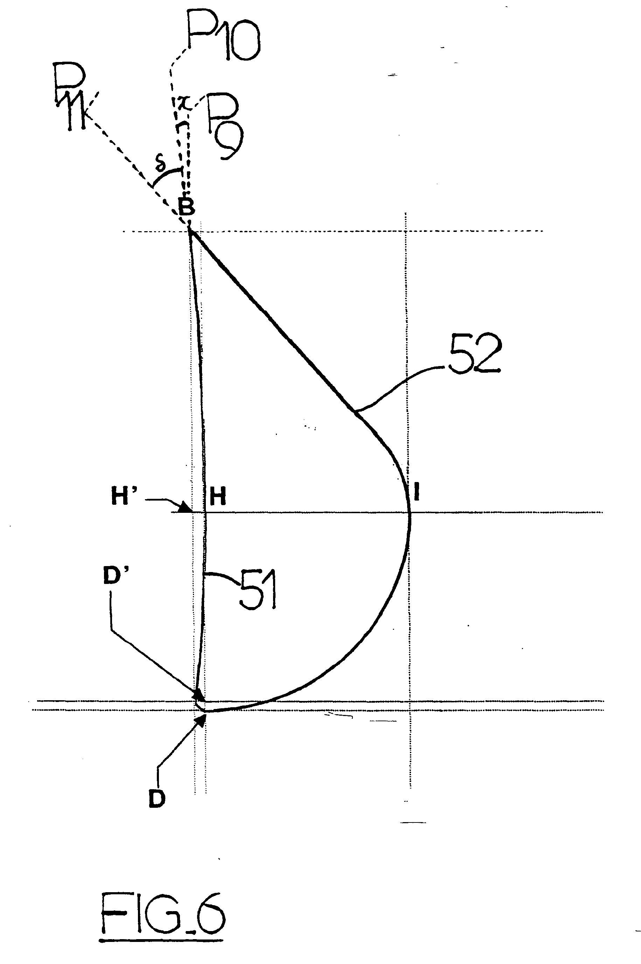

La figure 6 est une vue de profil de la prothèse 31. Le point I est le pôle dé la face antérieure 52 selon le plan de la figure. Le point H est le pôle de la face postérieure 51 selon le plan de la figure. Le point H' est la projection perpendiculaire de H sur un plan P9 vertical passant par B et perpendiculaire au plan de la figure. Les distances entre ces différents points sont les suivantes :
HH' = 3,5 mm
HI = 5 cm (projection antérieure de la prothèse)
HD = 5 cm
H'B = 7 cm
DD' = 2 mm

La face postérieure 51 présente une seconde concavité dans le plan de la figure. Cette concavité peut se quantifier par la distance HH' qui est supérieure à 1 mm, et par les angles χ que fait le plan tangeant à la face postérieure 51 au point B avec le plan P9. (La situation est la même au point D', la concavité s'étendant de B jusqu'à D'). Ici , l'angle χ est d'environ 7°, et peut être compris par exemple entre 4° et 15°.

La figure 6 permet aussi de voir un second raccordement en pente douce, sur la zone supérieure de la prothèse : au point B, l'angle δ que fait le plan P10 explicité plus haut et le plan P11 tangeant à la face antérieure au point B est faible, bien inférieur à 90° ou à 60°, et il est choisi ici d'environ 38.5°.

La distance HI est une caractéristique importante de la prothèse car elle permet de définir la projection antérieure de la prothèse. Dans cet exemple précis elle est de 5 centimètres, mais on peut la choisir plus généralement dans une gamme allant de 3 à 7 centimètres.

En conclusion, cet exemple de prothèse non limitatif est celle qui combine toutes les caractéristiques de l'invention pour se rapprocher bien mieux qu'auparavant de l'aspect du sein naturel. Des prothèses de volume différents peuvent se déduire par simple homothétie. Cependant, il reste dans le cadre de l'invention de prévoir des prothèses qui ne cumuleraient pas l'ensemble des moyens de latéralisation, (adaptation à la convexité thoracique par une face postérieure concave et/ou au moins un « raccordement en pente douce », et/ou dissymétrie par rapport à un plan vertical passant par le mamelon,et/ou « débordement « externe...).

## Revendications

1. Prosthèse mammaire implantable, destinée en particulier à la chirurgie mammaire reconstructrice et d'augmentation et comportant une poche souple (2) susceptible de contenir un gel de silicone, un hydrocolloide ou un sérum physiologique comme matériau de remplissage suffisamment fluide (3), ladite prothèse (1) étant latéralisée et les plans P7 et P8 tangeant respectivement à la face antérieure (52) et à la face postérieure (51) de la poche (2) une fois remplie et en position d'implantation au bord interne (C) faisant un angle β inférieur à 70°,
**caractérisée en ce que** la face postérieure (51) de la poche (2) en position d'implantation présente une courbure concave dans un plan vertical (P9) passant notamment par le bord supérieur (B), la distance (HH') entre le pôle (H) de la face postérieure (51) selon la courbure concave dans le plan vertical et le plan (P9) vertical passant par le bord supérieur (B), distance mesurée perpendiculairement audit plan (P9), étant d'au moins 1 mm, notamment d'au moins 2 mm et de préférence comprise entre 3 et 6 mm.

2. Prothèse selon la revendication 1, **caractérisée en ce que** la face postérieure (51) de la poche (2) en position d'implantation présente une courbure concave dans un plan horizontal (P3), passant notamment par le bord interne (C)

3. Prothèse selon la revendication 2, **caractérisée en ce que** la projection GG' du pôle (G) de la face postérieure (51) selon la courbure concave dans un plan horizontal sur le plan (P4) horizontal et contenant les bords interne (C) et postérieur externe (A'), distance mesurée perpendiculairement audit plan, est d'au moins 5 mm, notamment d'au moins 1 cm.

4. Prothèse selon l'une des revendications précédentes, **caractérisée en ce que** la poche (2) une fois remplie et en position d'implantation présente un débordement externe (44) par rapport à sa face postérieure (51), s'étendant notamment entre les bords postérieurs inférieur (D) et supérieur (B), le plan (P5) tangent au point k au bord postérieur externe à la face antérieure (52) faisant avec le plan P6, audit point k, à la face postérieure (51) un angle obtus (Φ) compris entre 91° et 120°, par exemple de l'ordre de 115°.

5. Prothèse selon l'une des revendications précédentes, **caractérisée en ce que** le rapport r(EC/EA) entre les projections (EC, EA) selon un plan horizontal (P2) contenant le mamelon (E) des distances respectives entre le mamelon (E) et les bords antérieur interne (C) et antérieur externe (A) est compris entre 0,8 et 0,9 et de préférence est égal à environ 0,875, et **en ce que** selon le plan (P2), la dimension de la projection selon le plan (EC) de la distance entre le mamelon (E) et le bord interne (C) de la dimension de la projection (EA') de la distance entre ledit mamelon (E) et le bord postérieur externe (A') sont égales ou très voisines.

6. Prothèse selon l'une des revendications précédentes, **caractérisée en ce que** le rapport r(BE/ED) entre les projections (BE, ED) selon un plan vertical (P1) contenant le mamelon (E) des distances respectives entre le mamelon (E) et les bords supérieur (B) et inférieur (D) est d'au moins 1,1, notamment entre 1,1 et 2 et de préférence entre 1,3 et 1,5.

7. Prothèse selon l'une des revendications précédentes, **caractérisée en ce que** la projection de la distance (HI) entre le pôle (H) de la face postérieure (51) et le pôle (I) de la face antérieure (52) selon un plan vertical passant par les bords supérieur (B) et inférieur (D) est comprise entre 3 et 7 centimètres et de préférence voisin de 5 centimètres.

8. Prothèse selon l'une des revendications précédentes, **caractérisée en ce qu'**au moins une partie de la face postérieure (51) de la poche est moins déformable ou plus rigide que le reste de ladite poche (2), notamment par épaississement sélectif de ladite face postérieure.

9. Prothèse selon l'une des revendications précédentes, **caractérisée en ce que** les plans (P7, P8) tangents respectivement à la face antérieure (52) et à la face postérieure (51) de la poche (2) une fois remplie et en position d'implantation au bord interne (C) font un angle (β) inférieur à 65°, notamment inférieur à 60°, de préférence voisin de 40°.

10. Prothèse selon l'une des revendications précédentes, **caractérisée en ce que** les plans (P11, P11) tangents respectivement à la face postérieure (51) et à la face antérieure (52) de la poche (2) une fois remplie et en position d'implantation au bord supérieur (B) font un angle (δ) inférieur à 70°, notamment inférieur à 65 ou 60°, de préférence d'environ 40°.

11. Prothèse selon l'une des revendications précédentes, **caractérisée en ce que** la poche (2) est remplissable par le matériau de remplissage avant et/ou pendant et/ou après l'implantation chirurgicale.

## Claims

1. Implantable mastectomy prosthesis, intended in particular for mammary surgical reconstruction and enlargement and comprising a flexible pocket (2) able to contain a silicone gel, a hydrocolloid or a physiological serum as sufficiently fluid filling material (3), aforementioned prosthesis (1) being lateralized and planes P7 and P8 respectively tangent to anterior face (52) and posterior face (51) of pocket (2) when filled and in the implantation position at internal edge (C) forming an angle β of less than 70°, **characterized in that** posterior face (51) of pocket (2) when in the implantation position presents a concave curve in a vertical plane (P9) passing in particular through the upper edge (B); distance (HH') between pole (H) of posterior face (51) according to the concave curve in the vertical plane and vertical plane (P9) passing through upper edge (B) measured perpendicular to plane (P9) being at least 1 mm, and in particular at least 2 mm and preferably between 3 and 6 mm.

2. Prosthesis according to claim 1, **characterized in that** posterior face (51) of pocket (2) when in the implantation position presents a concave curve in a horizontal plane (P3) passing in particular through internal edge (C)

3. Prosthesis according to claim 2, **characterized in that** projection GG' of posterior face (51) from pole (G) according to the concave curve in a horizontal plane on horizontal plane (P4) and containing internal edge (C) and external posterior edge (A'); the distance measured perpendicular to the said plan being at least 5 mm, and in particular at least 1 cm.

4. Prosthesis according to one of the preceding claims, **characterized in that** once filled and in the implantation position, pocket (2) presents an external extension (44) relative to its posterior face (51), extending in particular between lower posterior edge (D) and upper posterior edge (B), plane (P5), tangent to the posterior edge external to anterior face (52) at point K forming with plane P6, to posterior face (51), at said point K, an obtuse angle (φ) of between 91° and 120°, for example about 115°.

5. Prosthesis according to one of the preceding claims, **characterized in that** the ratio r(EC/EA) between projections (EC, EA), according to a horizontal plane (P2) containing nipple (E), of the distances between nipple (E) and internal anterior edge (C) and anterior external edge (A) respectively is between 0.8 and 0.9 and preferably equal to approximately 0.875, and **in that** according to plane (P2), the projection dimension according to plane (EC) of the distance between nipple (E) and internal edge (C) and the projection dimension (EA') of the distance between aforementioned nipple (E) and external posterior edge (A') are equal or very similar.

6. Prosthesis according to one of the preceding claims, **characterized in that** ratio r(BE/ED) between projections (BE, ED) according to a vertical plane (P1) containing nipple (E) of the distances between nipple (E) and upper edge (B) and lower edge (D) respectively is at least 1.1, and in particular between 1.1 and 2 and preferably between 1.3 and 1.5.

7. Prosthesis according to one of the preceding claims, **characterized in that** the projections of distance (HI) between pole (H) of posterior face (51) and pole (I) of anterior face (52) along a vertical plane passing through upper edge (B) and lower edge (D) is between 3 and 7 centimeters and preferably close to 5 centimeters.

8. Prosthesis according to one of the preceding claims, **characterized in that** at least part of posterior face (51) of the pocket is less deformable or more rigid than the remainder of the aforesaid pocket (2), in particular by selective thickening of the aforesaid posterior face.

9. Prosthesis according to one of the preceding claims, **characterized in that** planes (P7, P8) respectively tangents of anterior face (52) and posterior face (51) of pocket (2) once filled and in the implantation position at the inside edge (C) form an angle (β) less than 65°, in particular less than 60° and preferably close to 40°.

10. Prosthesis according to one of the preceding claims, **characterized in that** planes (P11, P11) respectively tangents of posterior face (51) and anterior face (52) of pocket (2) once filled and in the implantation position at upper edge (B) form an angle (δ) of less than 70°, in particular less than 65° or 60° and preferably approximately 40°.

11. Prosthesis according to one of the preceding claims, **characterized in that** pocket (2) can be filled by the fill material before and/or during and/or after surgical implantation.

## Patentansprüche

1. Implantierbare Brustprothese bestimmt insbesondere zur wiederherstellenden und vergrößernden Brustchirurgie mit einer weichen Hülle (2), die mit einem Silicongel, einem Hydrocolloid oder einem physiologischem Serum als ausreichend fluidem (3) Inhaltsstoff gefüllt sein kann, wobei die genannte Prothese (1) lateralisiert ist und die Ebenen P7 und P8 als Tangente an die Vorderseite (52) bzw. die Rückseite (53) der befüllten und in Implantationsposition befindlichen Hülle (2) unter einem Winkel β kleiner 70° am inneren Rand (C) anliegen,
**gekennzeichnet dadurch, dass** die Rückseite (51) der Hülle (2) in Implantationsposition in einer senkrechten Ebene (P9) konkav gewölbt ist, die insbesondere durch den oberen Rand (B) geht, wobei die rechtwinklig zur genannten Ebene (P9) gemessene Entfernung mindestens 1 mm, insbesondere mindestens 2 mm und vorzugsweise zwischen 3 und 6 mm beträgt.

2. Prothese gemäss Anspruch 1, **gekennzeichnet dadurch, dass** die Rückseite (51) der Hülle (2) in Implantationsposition eine konkave Wölbung in horizontaler Ebene (P3) aufweist, die insbesondere durch den inneren Rand (C) geht.

3. Prothese gemäss Anspruch 2, **gekennzeichnet dadurch, dass** die Projektion GG' des Pols (G) der Rückseite (51), gemäss der konkaven Wölbung in einer horizontalen Ebene auf die horizontale Ebene (P4), in welcher der innere Rand (C) und der rückseitige äußere Rand (A') liegen, wobei die Entfernung senkrecht zur genannten Ebene gemessen wird, mindestens 5 mm, insbesondere mindestens 1 cm beträgt.

4. Prothese gemäss einem der vorstehenden Ansprüche, **gekennzeichnet dadurch, dass** die Hülle (2) nach dem Befüllen und in Implantationsposition in bezug auf ihre Rückseite (51) einen Überstand (44) nach außen aufweist, der sich insbesondere zwischen dem unteren (D) und dem oberen (B) rückseitigen Rand erstreckt, und dass die Ebene (P5), die im Punkt k dem äußeren rückwärtigen Rand der Rückseite (52) als Tangente anliegt, mit der Ebene P6 im genannten Punkt k, an der Rückseite (51), einen offenen Winkel Φ zwischen 91° und 120° bildet, zum Beispiel etwa 115°.

5. Prothese gemäss einem der vorstehenden Ansprüche, **gekennzeichnet dadurch, dass** das Verhältnis (EC/EA) zwischen den Projektionen (EC, EA), gemäss einer horizontalen Ebene (P2), in der die Brustwarze liegt, der Entfernungen zwischen der Brustwarze (E) und dem vorderen inneren Rand (C) sowie dem rückwärtigen äußeren Rand (A) zwischen 0,8 und 0,9, vorzugsweise etwa 0,875 beträgt, und dass das Maß der Projektion gemäss Ebene (EC) der Entfernung zwischen der Brustwarze (E) und dem inneren Rand (C) sowie das Maß der Projektion (EA') der Entfernung zwischen der Brustwarze (E) und dem hinteren äußeren Rand (A') gleich oder fast gleich sind.

6. Prothese gemäss einem der vorstehenden Ansprüche, **gekennzeichnet dadurch, dass** das Verhältnis (BE/ED) zwischen den Projektionen (BE, ED), gemäss einer senkrechten Ebene (PI), in der die Brustwarze (E) liegt, der Entfernung zwischen der Brustwarze (E) und dem oberen (B) bzw. dem unteren (D) Rand mindestens 1,1, insbesondere zwischen 1,1 und 2, vorzugsweise zwischen 1,3 und 1,5 beträgt.

7. Prothese gemäss einem der vorstehenden Ansprüche, **gekennzeichnet dadurch, dass** die Projektion der Entfernung (HI) zwischen dem Pol (H) der Rückseite (51) und dem Pol (I) der Vorderseite (52) gemäss einer senkrechten Ebene, die durch den oberen (B) und unteren (D) Rand geht, zwischen 3 und 7 cm, vorzugsweise etwa 5 cm beträgt.

8. Prothese gemäss einem der vorstehenden Ansprüche, **gekennzeichnet dadurch, dass** mindestens ein Teil der Rückseite (51) der Hülle weniger verformbar d.h. steifer ist, als der Rest der genannten Hülle (2), insbesondere durch eine selektive Verdickung der genannten Rückseite.

9. Prothese gemäss einem der vorstehenden Ansprüche, **gekennzeichnet dadurch, dass** die Ebenen (P7, P8), die als Tangenten der Vorderseite (52) oder der Rückseite (51) der Hülle (2) anliegen, nach dem Befüllen und in Implantationsposition, mit dem inneren Rand (C) einen Winkel (P) von kleiner 65°, insbesondere von kleiner 60°, vorzugsweise nahe 40° bilden.

10. Prothese gemäss einem der vorstehenden Ansprüche, **gekennzeichnet dadurch, dass** die Ebenen (PI 1, PI 1), welche die Vorderseite (52) oder die Rückseite (51) der Hülle (2) berühren, nach dem Befüllen und in Implantationsposition mit dem inneren Rand (C) einen Winkel (P) von kleiner 70°, insbesondere von kleiner 60°, vorzugsweise nahe 40° bilden.

11. Prothese gemäss einem der vorstehenden Ansprüche, **gekennzeichnet dadurch, dass** die Hülle (2) vor und/oder während der chirurgischen Implantation mit dem Füllmaterial befüllbar ist.
